# EUROPEAN PATENT SPECIFICATION

(11) **EP 2 036 538 B1**
(45) Date of publication and mention of the grant of the patent: **22.06.2011**
(21) Application number: 08380004.5
(22) Date of filing: 10.01.2008
(51) Int. Cl.: A61K 9/00, A61K 31/535, A61K 31/498, A61K 31/382

(54) **Pharmaceutically stable compound consisting of timolol, dorzolamide and brimonidine**
Pharmazeutisch stabile Verbindung aus Timolol, Dorzolamid und Brimonidin
Composant stable pharmaceutique à base de timolol, de dorzolamide et brimonidine

(30) Priority: 12.09.2007 MX MX07011165
(43) Date of publication of application: 18.03.2009
(73) Proprietor: Jimenez Bayardo, Arturo, Guadalajara, Jalisco 44100 (MX)
(72) Inventor: Tornero Montaño, Jose Ruben, Guadalajara Jalisco 44100 (MX); Baiza Duran, Leopoldo Martin, Guadalajara Jalisco 44100 (MX); Quintana Hau, Juan de Dios, Guadalajara Jalisco 44100 (MX)
(74) Representative: Temino Ceniceros, Ignacio

(56) References cited:
- WO-A-2007/121077
- US-A1- 2004 029 771
- US-A1- 2005 038 103
- NOECKER R J ET AL: "CORNEAL AND CONJUCTIVAL CHANGES CAUSED BY COMMONLY USED GLAUCOMA MEDICATIONS" CORNEA, MASSON PUBL., NEW YORK, NY, US, vol. 23, no. 5, 1 July 2004 (2004-07-01), pages 490-496, XP009069206 ISSN: 0277-3740

## Description

### FIELD OF THE INVENTION

The present invention is related to ophthalmic formulations for the treatment of ocular ailments. More specifically, it refers to a formulation in a Timolol Maleate, Dorzolamide Hydrochloride and Brimonidine Tartrate based solution, which contains a combination of a tonifying agent, a buffer agent and a solubilizing agent and a preservative agent and which may be characterized by having excellent stability properties. More specifically, the present invention is related to the pharmaceutical industry and the production of ophthalmic medicine for the treatment of ocular hypertension.

### BACKGROUND OF THE INVENTION

The combination of Dorzolamide Hydrochloride, Timolol Maleate and Brimonidine Tartrate has proven to have significant advantages over state of the art ocular hypertension agents.

It is well known that pharmaceutical compositions in general are made up of excipients with diverse functions.

In the case of ophthalmic medications, for external use on the eye, the products are generally liquid solutions.

In order to obtain a stable formula, it is important to consider various excipients which are necessary to obtain chemical stability, preventing the active ingredients which make up the excipient from degrading or reacting among themselves, such as a pH buffer, a preservative, an agent which regulates the osmolarity, a solubilizing agent, etc.

That is to say, the excipients are a combination of a tonifying agent, a buffering agent, a solubilizing agent and a preservative agent.

Notwithstanding this knowledge within the state of the art, pharmaceutical research and development are necessary in order to determine which specific excipients may be used in a product in combination with the three molecules in question.

Mannitol is widely used in pharmaceutical formulas and food. It is mainly used in pharmaceutical preparations as a diluent in tablets and in liquid solutions to adjust the osmolarity.

Therapeutically Mannitol which is parenterally administered is used as an osmotic diuretic, as a diagnostic agent of kidney function, as well as being used to reduce intracranial pressure, in the treatment of cerebral edema and to reduce intraocular pressure.

Mannitol is present in almost all vegetables. It acts as a laxative if consumed in large amounts. Following an intravenous injection, Mannitol is not metabolized and is only absorbed slightly in the renal tract, thus about 80% of the dose is excreted in urine in the first 3 hours.

Polyoxyl 40 stearate is generally used as an emulsifier in oily/water type creams and lotions. Polyoxyl 40 stearate has been used as an emulsifying agent in intravenous infusions.

Although Polyoxyethylenes are mainly used as emulsifying agents in pharmaceutical formulas for external use, certain materials, specifically Polyoxyl 40 stearate, have also been used in intravenous injections and oral preparations.

Polyoxyethylenes stearates have been extensively tested for toxicity in animals and are widely used in pharmaceutical and cosmetic formulations.

They are generally classified as non-toxic and non-irritant materials.

The combination of Boric Acid and Sodium Borate possesses good buffering ability and is commonly used in ophthalmic preparations as pH buffers.

Sodium Chloride is widely used in a variety of parenteral and non-parenteral pharmaceutical formulations, where the principle purpose is to produce isotonic solutions. In ophthalmic formulations the main use is to regulate osmolarity.

Sodium Chloride is the most important salt found in the body; it regulates osmotic tension in the blood and tissues. Approximately 5 to 12 g of Sodium Chloride are consumed daily by an individual on a normal diet and the same amount is excreted in urine every day. As an excipient Sodium Chloride may be added as a non-toxic and non-irritant material.

Benzalkonium chloride is a component of quaternary ammonium which is used in pharmaceutical formulations as a preservative in similar applications to other cationic surfactant agents such as Cetrimide.

In ophthalmic formulations Benzalkonium chloride is one of the most widely used preservatives, used in concentrations of between 0.01 and 0.02 %.

Benzalkonium chloride is usually not irritating and is well tolerated in the concentrations normally used on the skin and mucose. However, Benzalkonium chloride has been associated with adverse effects in some ophthalmic formulations. Toxicity experiments on rabbits show that Benzalkonium chloride is damaging in concentrations above those which are normally used in formulations. However, the human eye seems to be less affected than the eye of a rabbit.

To date, there is no known ophthalmic formulation which specifically suggests the combination of Dorzolamide Hydrochloride, Timolol Maleate and Brimonidine Tartrate for the treatment of ocular hypertension, and thus the present invention has inventive merits of its own as will become evident through the following description.

### OBJECTIVES OF THE INVENTION

One of the objectives of the invention is to achieve a composition of Dorzolamide Hydrochloride, Timolol Maleate and Brimonidine Tartrate which is phisiochemically compatible and stable.

Another objective is to determine the qualitative composition of the excipients which achieve the previous objective.

Still another objective is to determine whether, in the combinations which achieve the first objective, chemical reactions occur which produce modifications in the active molecules.

Yet another objective of the present invention is to demonstrate that there is no antagonistic effect among the components.

All of the previous objectives will become apparent through the following description and the annexes.

### BRIEF DESCRIPTION OF THE INVENTION

The present invention consists of a qualitative composition as well as a novel quantitative composition for the treatment of ocular hypertension containing a combination of Dorzolamide Hydrochloride, Timolol Maleate and Brimonidine Tartrate, with excipients which allow for the co-existence of the three active principles with good stability.

Following multiple efforts of selection, it was determined that base composition of the excipients should contain at least the following components: Polyoxyl 40 stearate, Sodium Borate crystals, Sodium chloride and Benzalkonium chloride.

In one of the preferred incorporations, Mannitol is included in the composition of the previous base excipients.

In another of the preferred incorporations Hydroxypropyl-beta-cyclodextrines and sodium hyaluronate are added to the qualitative composition of base excipients.

In general, one of the aspects of the invention consists of a composition of Dorzolamide Hydrochloride, Timolol Maleate and Brimonidine Tartrate, with the following quantitative composition:

| **Component** | **Amount (g)** |
|---|---|
| Polyoxyl 40 Stearate | 5.0-7.0 |
| Sodium Borate crystals | 0.34-0.56 |
| Sodium Chloride | 0.10-0.18 |
| Benzalkonium chloride | 0.02-0.022 |
| Mannitol | 0.0-0.50 |
| Hydroxypropyl-beta-cyclodextrines | 0.0-1.0 |
| Sodium Hyaluronate | 0.0-0.20 |
| Timolol Maleate | 0.68 |
| Brimonidine Tartrate | 0.20 |
| Dorzolamide Hydrochloride | 2.22 |
| Water as a vehicle | 100.0 ml |

Based on the results of tests of excipient compatibility, the base was obtained for carrying out the following formulas which underwent accelerated stability during 3 months (40°C) in low density polyethylene jars.

| **Component** | **Amount (g)** |
|---|---|
| Polyoxyl 40 Stearate | 5.0-7.0 |
| Sodium Borate crystals | 0.34-0.56 |
| Sodium Chloride | 0.10-0.18 |
| Benzalkonium chloride | 0.02-0.022 |
| Timolol Maleate | 0.68 |
| Brimonidine Tartrate | 0.20 |
| Dorzolamide Hydrochloride | 2.22 |
| Water as a vehicle | 100.0 ml |

There is a second formula with the following content:

| **Component** | **Amount (g)** |
|---|---|
| Polyoxyl 40 Stearate | 5.0-7.0 |
| Sodium Borate crystals | 0.34-0.56 |
| Sodium Chloride | 0.10-0.18 |
| Benzalkonium chloride | 0.02-0.022 |
| Mannitol | 0.50 |
| Timolol Maleate | 0.68 |
| Brimonidine Tartrate | 0.20 |
| Dorzolamide Hydrochloride | 2.22 |
| Water as a vehicle | 100.0 ml |

And finally a 3^{rd} formula with the following content:

| Component | Amount (g) |
|---|---|
| Polyoxyl 40 Stearate | 5.0-7.0 |
| Sodium Borate crystals | 0.34-0.56 |
| Sodium Chloride | 0.10-0.18 |
| Benzalkonium chloride | 0.02-0.022 |
| Hydroxypropyl-beta-cyclodextrines | 1.0 |
| Sodium Hyaluronate | 0.20 |
| Timolol Maleate | 0.68 |
| Brimonidine Tartrate | 0.20 |
| Dorzolamide Hydrochloride | 2.22 |
| Water as a vehicle | 100.0 ml |

In order to better understand the invention, following is a detailed description of the present invention showing the results of various tests carried out with the chosen composition.

### DETAILED DESCRIPTION OF THE INVENTION

Following is a detailed description of the two facets of the present invention:

Following multiple efforts to decide, the base compound of the excipients should include at least the following components: a solubilizing agent such as Polyoxyl 40 Stearate, an agent which helps to buffer the pH such as Sodium Borate crystals, a tonifying agent such as Sodium Chloride and an antimicrobial preservative such as Benzalkonium chloride.

In one of the preferred incorporations, another tonifying agent is added to the composition of the base compound, that being Mannitol.

In another preferred incorporation Hydroxypropyl-beta-cyclodextrines and Sodium Hyaluronate are added to the qualitative composition of the base compounds.

Based on the results of compatibility tests of the excipients, the base needed to carry out the following formulas was found, these underwent accelerated stability during 3 months (40°) in low density polyethylene jars.

| Component | Amount (g) |
|---|---|
| Polyoxyl 40 Stearate | 5.0-7.0 |
| Sodium Borate crystals | 0.34-0.56 |
| Sodium Chloride | 0.10-0.18 |
| Benzalkonium chloride | 0.02-0.022 |
| Timolol Maleate | 0.68 |
| Brimonidine Tartrate | 0.20 |
| Dorzolamide Hydrochloride | 2.22 |
| Water as a vehicle | 100.0 ml |

A second formula with the following content was obtained:

| Component | Amount(g) |
|---|---|
| Polyoxyl 40 Stearate | 5.0-7.0 |
| Sodium Borate crystals | 0.34-0.56 |
| Sodium Chloride | 0.10-0.18 |
| Benzalkonium chloride | 0.02-0.022 |
| Mannitol | 0.50 |
| Timolol Maleate | 0.68 |
| Brimonidine Tartrate | 0.20 |
| Dorzolamide Hydrochloride | 2.22 |
| Water as a vehicle | 100.0 ml |

And finally a third formula with the following content:

| Component | Amount (g) |
|---|---|
| Polyoxyl 40 Stearate | 5.0-7.0 |
| Sodium Borate crystals | 0.34-0.56 |
| Sodium Chloride | 0.10-0.18 |
| Benzalkonium chloride | 0.02-0.022 |
| Hydroxypropyl-beta-cyclodextrines | 1.0 |
| Sodium Hyaluronate | 0.20 |
| Timolol Maleate | 0.68 |
| Brimonidine Tartrate | 0.20 |
| Dorzolamide Hydrochloride | 2.22 |
| Water as a vehicle | 100.0 ml |

Tests were carried out on 5 people to determine the level of stinging. Following are the results:

| ***1: NULL*** | ***2: SLIGHT*** | ***3: INTENSE*** | | | |
|---|---|---|---|---|---|
| | **PERSON 1** | **PERSON 2** | **PERSON 3** | **PERSON 4** | **PERSON 5** |
| Formula 1 | 3 | 3 | 3 | 3 | 3 |
| Formula 2 | 2 | 2 | 2 | 2 | 2 |
| Formula 3 | 2 | 2 | 2 | 2 | 2 |

Based on the preceding results, only formulae 2 and 3 underwent accelerated stability.

Following are the results of the tests for accelerated stability:

### TIMOLOL MALEATE

| **FORMULA** | **INITIAL*** | **1 MONTH** | **2 MONTHS** | **3 MONTHS** |
|---|---|---|---|---|
| **2** | 100.0 | 101.01 | 99.89 | 102.73 |
| **3** | 100.0 | 99.59 | 101.20 | 103.72 |

| | | | | |
|---|---|---|---|---|
| *Results normalized at 100 %. | | | | |

### BRIMONIDINE TARTRATE

| **FORMULA** | **INITIAL*** | **1 MONTH** | **2 MONTHS** | **3 MONTHS** |
|---|---|---|---|---|
| **2** | 100.0 | 96.25 | 98.23 | 103.31 |
| **3** | 100.0 | 110.00 | 96.80 | 102.14 |

| | | | | |
|---|---|---|---|---|
| *Results normalizaded at 100 %. | | | | |

### DORZOLAMIDE HYDROCHLORIDE

| **FORMULA** | **INITIAL*** | **1 MONTH** | **2 MONTHS** | **3 MONTHS** |
|---|---|---|---|---|
| **2** | 100.0 | 101.79 | 103.64 | 104.69 |
| **3** | 100.0 | 124.0 | 99.11 | 104.82 |

| | | | | |
|---|---|---|---|---|
| *Results normalized at 100 %. | | | | |

Based on the results of stability, Formula F 2 was chosen due to its chemical stability and less stinging.

That is, in one of its preferred incorporations, the invention consists of:

| | | |
|---|---|---|
| Polyoxyl 40 Stearate | | 7.00 g |
| Sodium Borate crystals | | 0.56 g |
| Sodium Chloride | | 0.10 g |
| Benzalkonium chloride | | 0.02 g |
| Mannitol | | 0.50 g |
| Timolol Maleate | | 0.68 g |
| Brimonidine Tartrate | | 0.20 g |
| Dorzolamide Hydrochloride | | 2.22 g |
| Water as a vehicle | | 100.0 mL |
| | pH= 5.65. | |

Having established this as the optimum formula, certain tests were carried out to find the functional intervals of the components, finding the following intervals to be the most appropriate:

| | | |
|---|---|---|
| Polyoxyl 40 Stearate | | 6.15-8.00 g |
| Sodium Borate crystals | | 0.48-0.65 g |
| Sodium Chloride | | 0.09-0.12 g |
| Benzalkonium chloride | | 0.02-0.023 g |
| Mannitol | | 0.35-0.85 g |
| Timolol Maleate | | 0.68 g |
| Brimonidine Tartrate | | 0.20 g |
| Dorzolamide Hydrochloride | | 2.22 g |
| Water as a vehicle | | 100.0 mL |
| | pH= 5.65. | |

The invention has been sufficiently described so that a person with average knowledge in the field may reproduce and obtain the results which we mention in the present invention.

## Claims

1. A stable pharmaceutical composition for the treatment of ocular hypertension of the kind that consists of the active molecules Timolol Maleate, Brimonidine Tartrate and Dorzolamide Hydrochloride, **characterized by** fundamentally consisting of the following excipients: Polyoxyl 40 Stearate, Sodium Borate crystals, Sodium Chloride and Benzalkonium chloride.

2. A stable pharmaceutical composition for the treatment of ocular hypertension, as claimed in the preceding claim, **characterized by** also consisting of the excipient Mannitol.

3. A stable pharmaceutical composition for the treatment of ocular hypertension, as claimed in Claim 1, **characterized by** also consisting of the excipients: Hydroxypropyl-beta-cyclodextrines and Sodium Hyaluronate.

4. A stable pharmaceutical composition for the treatment of ocular hypertension, as claimed in Claims 1, 2 and 3, **characterized by** consisting of the following quantitative composition:
| Component | Amount (g) |
|---|---|
| Polyoxyl 40 Stearate | 5.0-7.0 |
| Sodium Borate crystals | 0.34-0.56 |
| Sodium Chloride | 0.10-0.18 |
| Benzalkonium chloride | 0.02-0.022 |
| Mannitol | 0.0-0.50 |
| Hydroxypropyl-beta-cyclodextrines | 0.0-1.0 |
| Sodium Hyaluronate | 0.0-0.20 |
| Timolol Maleate | 0.68 |
| Brimonidine Tartrate | 0.20 |
| Dorzolamide Hydrochloride | 2.22 |
| Water as a vehicle | 100.0 ml |

5. A stable pharmaceutical composition for the treatment of ocular hypertension, as claimed in Claim 2, **characterized by** consisting of a quantitative composition with the following content:
| | | |
|---|---|---|
| Polyoxyl 40 Stearate | 6.15-8.00 | g |
| Sodium Borate crystals | 0.48-0.65 | g |
| Sodium Chloride | 0.09-0.12 | g |
| Benzalkonium chloride | 0.02-0.023 | g |
| Mannitol | 0.35-0.85 | g |
| Timolol Maleate | 0.68 | g |
| Brimonidine Tartrate | 0.20 | g |
| Dorzolamide Hydrochloride | 2.22 | g |
| Water as a vehicle | 100.0 | mL |
| pH= 5.65. | | |

6. A stable pharmaceutical composition for the treatment of ocular hypertension, as claimed in the preceding claim, **characterized by** consisting of a quantitative composition with the following content:
| | | |
|---|---|---|
| Polyoxyl 40 Stearate | 7.00 | g |
| Sodium Borate crystals | 0.56 | g |
| Sodium Chloride | 0.10 | g |
| Benzalkonium chloride | 0.02 | g |
| Mannitol | 0.50 | g |
| Timolol Maleate | 0.68 | g |
| Brimonidine Tartrate | 0.20 | g |
| Dorzolamide Hydrochloride | 2.22 | g |
| Water as a vehicle | 100.0 | mL |
| pH= 5.65. | | |

## Patentansprüche

1. Eine stabile pharmazeutische Zusammensetzung für die Behandlung von okularer Hypertonie von der aus den aktiven Molekülen Timololmaleat, Brimonidintartrat und Dorzolamidhydrochlorid bestehtenden Art, **dadurch gekennzeichnet, dass** es im Wesentlichen aus den folgenden Hilfsstoffen besteht: Polyoxyl (40) Stearat, Natriumborat-Kristalle, Natriumchlorid und Benzalkoniumchlorid.

2. Eine stabile pharmazeutische Zusammensetzung für die Behandlung von okularer Hypertonie nach dem vorhergehenden Anspruch, **dadurch gekennzeichnet, dass** es auch aus dem Hilfsstoff Mannitol besteht.

3. Eine stabile pharmazeutische Zusammensetzung für die Behandlung von okularer Hypertonie nach Anspruch 1, **dadurch gekennzeichnet, dass** es auch aus den Hilfsstoffen: Hydroxypropyl-beta-Cyclodextrine und Natriumhyaluronat besteht.

4. Eine stabile pharmazeutische Zusammensetzung für die Behandlung von okularer Hypertonie nach einem der Ansprüche 1, 2 und 3, **dadurch gekennzeichnet, dass** es aus der folgenden quantitativen Zusammensetzung besteht:
| Komponente | Masse (g) |
|---|---|
| Polyoxyl (40) Stearat | 5,0-7,0 |
| Natriumborat-Kristalle | 0,34-0,56 |
| Natriumchlorid | 0,10-0,18 |
| Benzalkoniumchlorid | 0,02-0,022 |
| Mannitol | 0,0-0,50 |
| Hydroxypropyl-beta-Cyclodextrine | 0,0-1,0 |
| Natriumhyaluronat | 0,0-0,20 |
| Timololmaleat | 0,68 |
| Brimonidintartrat | 0,20 |
| Dorzolamidhydrochlorid | 2,22 |
| Wasser als ein Träger | 100,0 ml |

5. Eine stabile pharmazeutische Zusammensetzung für die Behandlung von okularer Hypertonie nach Anspruch 2, **dadurch gekennzeichnet, dass** es aus einer quantitativen Zusammensetzung mit dem folgenden Inhalt besteht:
| | |
|---|---|
| Polyoxyl (40) Stearat | 6,15-8,00 g |
| Natriumborat-Kristalle | 0,48-0,65 g |
| Natriumchlorid | 0,09-0,12 g |
| Benzalkoniumchlorid | 0,02-0,023 g |
| Mannitol | 0,35-0,85 g |
| Timololmaleat | 0,68 g |
| Brimonidintartrat | 0,20 g |
| Dorzolamidhydrochtorid | 2,22 g |
| Wasser als ein Träger | 100,0 ml |
| pH = 5,65. | |

6. Eine stabile pharmazeutische Zusammensetzung für die Behandlung von okularer Hypertonie nach dem vorhergehenden Anspruch, **dadurch gekennzeichnet, dass** es aus einer quantitativen Zusammensetzung mit dem folgenden Inhalt besteht:
| | |
|---|---|
| Polyoxyl (40) Stearat | 7.00 g |
| Natriumborat-Kristalle | 0.56 g |
| Natriumchlorid | 0.10 g |
| Benzalkoniumchlorid | 0.02 g |
| Mannitol | 0.50 g |
| Timololmaleat | 0.68 g |
| Brimonidintartrat | 0.20 g |
| Dorzolamidhydrochlorid | 2.22 g |
| Wasser als ein Träger | 100.0 ml |
| pH = 5,65. | |

## Revendications

1. Une composition pharmaceutique stable pour le traitement de l'hypertension oculaire de ce genre qui se compose des molécules actives maléate de timolol, tartrate de brimonidine et chlorhydrate de dorzolamide, **caractérisé en ce que**, fondamentalement, constitué par les excipients suivants: stéarate de polyoxyl 40, cristaux de borate de sodium, chlorure de sodium et chlorure de benzalkonium.

2. Une composition pharmaceutique stable pour le traitement de l'hypertension oculaire, tel que revendiqué dans la revendication précédente, **caractérisé en ce que** constitué également par l'excipient mannitol.

3. Une composition pharmaceutique stable pour le traitement de l'hypertension oculaire, selon la revendication 1, **caractérisé en ce que** constitué également par l'un des excipients: hydroxypropyl-bêta-cyclodextrines et de hyaluronate de sodium.

4. Une composition pharmaceutique stable pour le traitement de l'hypertension oculaire, selon les revendications 1, 2 et 3, **caractérisé en ce que** constitué par la composition quantitative suivante:
| Composant | Montant (g) |
|---|---|
| Stéarate de polyoxyl 40 | 5,0-7,0 |
| Cristaux de borate de sodium | 0,34-0,56 |
| Chlorure de sodium | 0,10-0,18 |
| Chlorure de benzalkonium | 0,02-0,022 |
| Mannitol | 0,0-0,50 |
| hydroxypropyl-béta-cyclodextrines | 0,0-1,0 |
| Hyaluronate de sodium | 0,0-0,20 |
| Maléate de timolol | 0,68 |
| Tartrate de brimonidine | 0,20 |
| Chlorhydrate de dorzolamide | 2,22 |
| L'eau comme un véhicule | 100,0 ml |

5. Une composition pharmaceutique stable pour le traitement de l'hypertension oculaire, selon la revendication 1, **caractérisé en ce que** constitué par une composition quantitative avec le contenu suivant:
| | |
|---|---|
| Stéarate de polyoxyl 40 | 6,15-8,00 g |
| Cristaux de borate de sodium | 0,48-0,65 g |
| Chlorure de sodium | 0,09-0,12 g |
| Chlorure de benzalkonium | 0,02-0,023 g |
| Mannitol | 0,35-0,85 g |
| Maléate de timolol | 0,68 g |
| Tartrate de brimonidine | 0,20 g |
| Chlorhydrate de dorzolamide | 2,22 g |
| L'eau comme un véhicule | 100,0 ml |
| pH = 5,65. | |

6. Une composition pharmaceutique stable pour le traitement de l'hypertension oculaire, tel que revendiqué dans la revendication précédente, **caractérisé en ce que** constitué par une composition quantitative avec le contenu suivant:
| | |
|---|---|
| Stéarate de polyoxyl 40 | 7,00 g |
| Cristaux de borate de sodium | 0,56 g |
| Chlorure de sodium | 0,10 g |
| Chlorure de benzalkonium | 0,02 g |
| Mannitol | 0,50 g |
| Maléate de timolol | 0,68 g |
| Tartrate de brimonidine | 0,20 g |
| Chlorhydrate de dorzolamide | 2,22 g |
| L'eau comme un véhicule | 100,0 ml |
| pH = 5,65. | |
